# EUROPEAN PATENT SPECIFICATION

(11) **EP 2 189 436 B1**
(45) Date of publication and mention of the grant of the patent: **16.01.2013**
(21) Application number: 09177028.9
(22) Date of filing: 25.11.2009
(51) Int. Cl.: C07C 4/04, C07C 11/04, C10G 9/14, C10G 9/36

(54) **A method of producing ethylene by using refinery C4**
Verfahren zur Herstellung von Ethylen unter Verwendung einer C4-Fraktion aus der Raffinierung
Procédé de production d'éthylène utilisant du C4 de raffinerie

(30) Priority: 25.11.2008 CN 200810227274
(43) Date of publication of application: 26.05.2010
(73) Proprietor: Petrochina Company Limited, Dongcheng District Beijing Beijing 100007 (CN)
(72) Inventor: He, Defu, Dongcheng District Bejing 100007 Beijing (CN); Wang, Sihan, Dongcheng District Bejing 100007 Beijing, (CN); Zhang, Baojun, Dongcheng District Bejing 100007 Beijing, (CN); Qu, Jiabo, Dongcheng District Bejing 100007 Beijing (CN); Zhang, Yongjun, Dongcheng District Bejing 100007 Beijing (CN); Meng, Rui, Dongcheng District Bejing 100007 Beijing (CN); Wan, Shubao, Dongcheng District Bejing 100007 Beijing (CN); Wang, Shulan, Dongcheng District Bejing 100007 Beijing (CN); Zhang, Jingyuan, Dongcheng District Bejing 100007 Beijing (CN); Zhao, Xiwu, Dongcheng District Bejing 100007 Beijing (CN); Guan, Weihong, Dongcheng District Bejing 100007 Beijing (CN); Feng, Yingjie, Dongcheng District Bejing 100007 Beijing (CN); Liu, Shuqing, Dongcheng District Bejing 100007 Beijing (CN); Yu, Xiangmin, Dongcheng District Bejing 100007 Beijing (CN); Ren, Hongbo, Dongcheng District Bejing 100007 Beijing (CN); Guo, Yingzi, Dongcheng District Bejing 100007 Beijing (CN); Zhao, Jingying, Dongcheng District Bejing 100007 Beijing (CN); He, Chunfeng, Dongcheng District Bejing 100007 Beijing (CN)
(74) Representative: Koepe & Partner

(56) References cited:
- DE-A1- 4 241 144
- GB-A- 1 481 766
- US-A- 4 492 624
- US-A- 4 997 525
- US-A1- 2006 089 518

## Description

### Field of the Invention

The invention relates to a method of producing ethylene, in particular to a method of producing ethylene by using the mixtures of refinery C4 and alkane-rich light hydrocarbon as raw materials through copyrolysis.

### Background of the Invention

Ethylene is the flagship product in the industry of petrochemical engineering and the basis for producing organic chemicals. The production scale, yield and techniques of ethylene mark the level of development of the industry of petrochemical engineering of a country. At present, naphtha is still the most main raw materials of ethylene pyrolysis in the world. With the growing shortage of naphtha resources, it is very essential to find the new raw materials of ethylene pyrolysis for the production of ethylene.

In recent years, with the rapid increase in the processing capacity of crude oil and the growing yield of ethylene in China, C4 resources as the petrochemical by-product are also expanding. The total production of C4 resources has exceeded 3000 kt/a, in which the production of C4 from oil refinery is up to 300-400 kt/a. Currently, the refinery C4 is mainly served as civil liquefied petroleum gas, and has very low value in use. How to sufficiently and reasonably make use of the refinery C4 resources, and develop the potential value of these part of C4 resoruces have attracted the interests of the researchers.

The research reports concerning C4 used as the raw materials of ethylene mainly focus on the catalytic cracking of C4, such as the Arco.'s Superflex technique and Lurge's Propylur technique. The catalytic cracking process needs to use cracking catalysts and has higher cost of production. Moreover, there is the questions involving the catalyst deactivation, and thus it is only suitable for small batch of production of ethylene. This technology is not suitable for dealing with the refinery C4 resources on a large scale.

The applicant of the present invention has ever investigated the thermal cracking behaviors of the refinery C4 as the raw material of ethylene. And it is found that the yield of ethylene is no more than 18% when refinery C4 is singly used as the raw materials to carry out the thermal cracking reaction. Additionally, coking in the tubes of cracking furnace is so severe that the cycle of operation thereof is badly affected. Based on this, the applicant further attempts to make use of the mixure of the refinery C4 and high performance light hydrocarbon as raw materials to undergo copyrolysis reation. Surprisingly, the yield of ethylene is greatly increased.

US 4 997 525 discloses a process for the preparation of olefins by the cracking of hydrocarbons consisting in passing a mixture of hydrocarbons and steam flowing in a cracking tube disposed inside a radiation zone of a furnace.

US 2006/089 518 relates to a method for steam-cracking naphtha comprising the passage in a steam cracker, in the presence of steam, of a feedstock of a paraffinic naphtha modified by the addition of the combination of a first component including a gasoline and of a second component including at least one of a hydrocarbon refinery gas and a feedstock rich in paraffins.

GB 1 481 766 describes a method for thermally steam cracking, in the presence of steam, a hydrocarbon charge stream including (i) a hydrocarbon component containing at least 9 carbon atoms, and (ii) a deisobutanized C3 to C5 paraffin stream, thereby forming a product stream containing (i) an ethylene fraction and (ii) a C3 to C4 olefin fraction.

DE 4 241 144 dicloses a process for the joint cracking of hydrocarbon charges, especially naphtha, and non hydrogenated C4 fractions.

US 4 492 624 relates to a method fro cracking heavy oils to olefins. In parallel streams, the heavy stream and a steam diluent are heated to the point of partial thermal cracking while in the other stream a lighter oil and steam are cracked to produce olefins.

### Summary of the Invention

The invention is aimed to provide a method of producing ethylene by using refinery C4, in which the mixture of alkane-rich light hydrocarbon and refinery C4 is selected as the raw materials of producing ethylene through copyrolysis, and the ratio between the light hydrocarbon and refinery C4 and the suitable process condition are determined.

The technical solution of the present invention is given as follows:

A method of producing ethylene by using refinery C4 comprising:
a refinery C4 as a first raw material which comprises 45%-55% isobutane, 5%-10% n-butane, 15%-20% isobutene, 10%-15% 1-butylene and 5%-10% 2-butylene based on the total weight percentage of the refinery C4, the weight average molecular weight thereof being 55-60,
an alkane-rich light hydrocarbon as a second raw material which comprises 40%-50% linear alkane, 25%-35% branched alkane, 15%-25% cyclane, less than or equal to 1% alkene and less than or equal to 10% arene based on the total weight percentage of the alkane-rich light hydrocarbon, the weight average molecular weight thereof being 90-100;
the refinery C4 being in amount of 10% to 30% of the total weight percentage of the refinery C4 and alkane-rich light hydrocarbon;
introducing the mixture of said first raw material and said second raw material into the upper part of the convection section of a cracking furnace, after diluted with steam, passing the diluted mixture through the lower part of the convection section to enter into the radiation section of said cracking furnace,
distributing the diluted mixture to manifolds to carry out copyrolysis reaction therein so as to produce ethylene,
wherein the cracking temperature is 840°C to 860°C, and the dilution ratio of steam/total raw materials is 0.30 to 0.60 wt/wt.

Preferably, the first raw material is in amount of 15%-20% of the total raw materials.

The term "copyrolysis", as used herein, refers to the pyrolysis of the mixture of the first raw material and the second raw material.

The term "refinery C4", as used herein, refers to the remaining intermediate fraction after the fraction above C3 and the fraction below C5 in the refinery gases (nitrogen, carbon monoxide, carbon dioxide, C₁, C₂.....C₆) produced during the process of catalytic cracking, catalytic reforming, hydrogen cracking and so on in oil refinery are removed by gas fractionating device.

According to the method provided in the present invention, the yield of ethylene attributed to the refinery C4 of the total raw materials can be greatly increased as compared with the separate cracking of refinery C4, while the cracking performance of light hydrocarbon is maintained. In this way, the use value of refinery C4 and also other C4-rich products can be effectively improved, thus notable economic benefits can be brought to the oil refinery.

### Brief Description of the Drawings

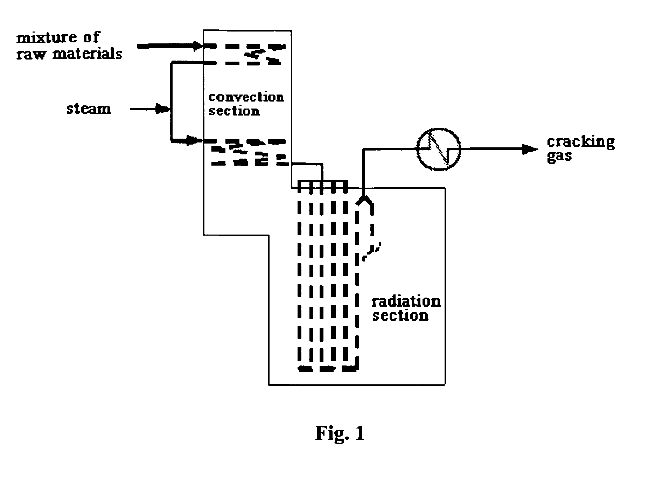

Fig. 1 is a schematic diagram showing the flow process in the cracking furnace.

### Detailed Description of the Invention

The following examples are set forth in order to further specify the invention. And it should be understood that these examples are only intended to be illustrative and not restrictive.

### Flow progress in the cracking furnace

As seen from figure 1, the mixture of refinery C4 as a first raw material and the alkane-rich light hydrocarbon as a second raw material are first introduced into the upper part of the convection section of a cracking furnace. Steam is used to dilute the mixture. And then the diluted mixture is passed through the lower part of the convection section to be further heated. After that, the diluted mixture flows into the radiation section of the cracking furnace, and is distributed to manifolds to carry out copyrolysis reaction therein. Thereafter, the cracking product is gathered together and rapidly cooled to creat the cracking gas in the outlet of cracking furnace.

### Measuring method for the yield of products

Sampling apparatus for cracking gas is utilized to collect samples in the outlet of the cracking furnace. And the samples are divided into gas samples and liquid samples. The gas samples is gauged by wet type gas flowmeter, and gas chromatography is used to analyze the composition of the end products. For the liquid samples, the fractions are cut to analyze the composition of the end products. Finally, the gas products and the liquid products are normalized by data processing to get the distribution of the yield of products in the outlet of the cracking furnace.

### Example 1

Raw materials used in the experiment: refinery C4 + alkane-rich light hydrocarbon, each composition of which is listed in table 1 and 2 below respectively, wherein the refinery C4 comprises 10 wt% of the total raw materials.

**Table 1 Composition of refinery C4**

| Composition | Content (wt %) |
|---|---|
| propane | 3.92 |
| cyclopropane | 1.23 |
| propene | 0.03 |
| isobutane | 46.36 |
| n-butane | 6.58 |
| trans-2-butylene | 4.10 |
| cis-2-butylene | 4.28 |
| 1-butylene | 12.06 |
| isobutene | 18.15 |
| others | 3.29 |
| Total | 100.00 |

**Table 2 Composition of alkane-rich light hydrocarbon**

| Composition | Content (wt %) |
|---|---|
| linear alkane | 44.28 |
| branched alkane | 28.35 |
| cyclane | 16.79 |
| alkene | 0.13 |
| arene | 8.66 |
| others | 1.79 |
| Total | 100.00 |

Experimental conditions: cracking temperature is 840°C, and the dilution ratio of steam/total raw materials is 0.30 wt/wt.

Cracking furnace type: USC-16W, available from Stone & Webster, Inc. of USA.

The results are as follows:

Ethylene yield: 29.59 wt%, propylene yield: 12.26 wt%, butadiene yield: 3.23 wt%.

### Example 2

Raw materials used in the experiment: refinery C4 + alkane-rich light hydrocarbon, each composition of which is listed in table 3 and 4 below respectively, wherein the refinery C4 comprises 10 wt% of the total raw materials.

**Table 3 Composition of refinery C4**

| Composition | Content (wt %) |
|---|---|
| propane | 2.38 |
| cyclopropane | 1.75 |
| propene | 0.05 |
| isobutane | 45.18 |
| n-butane | 5.36 |
| trans-2-butylene | 4.35 |
| cis-2-butylene | 5.51 |
| 1-butylene | 14.68 |
| isobutene | 19.21 |
| others | 1.53 |
| Total | 100.00 |

**Table 4 Composition of alkane-rich light hydrocarbon**

| Composition | Content (wt %) |
|---|---|
| linear alkane | 40.58 |
| branched alkane | 25.13 |
| cyclane | 24.35 |
| alkene | 0.12 |
| arene | 6.48 |
| others | 3.34 |
| Total | 100.00 |

Experimental conditions: cracking temperature is 840°C, and the dilution ratio of steam/total raw materials is 0.50 wt/wt.

Cracking furnace type: USC-16W, available from Stone & Webster, Inc. of USA.

The results are as follows:

Ethylene yield: 30.53 wt%, propylene yield: 11.65 wt%, butadiene yield: 3.25 wt%.

### Example 3

Raw materials used in the experiment: refinery C4 + alkane-rich light hydrocarbon, each composition of which is listed in table 5 and 6 below respectively, wherein the refinery C4 comprises 15 wt% of the total raw materials.

**Table 5 Composition of refinery C4**

| Composition | Content (wt %) |
|---|---|
| propane | 3.92 |
| cyclopropane | 1.23 |
| propene | 0.03 |
| isobutane | 46.36 |
| n-butane | 6.58 |
| trans-2-butylene | 4.10 |
| cis-2-butylene | 4.28 |
| 1-butylene | 12.06 |
| isobutene | 18.15 |
| others | 3.29 |
| Total | 100.00 |

**Table 6 Composition of alkane-rich light hydrocarbon**

| Composition | Content (wt %) |
|---|---|
| linear alkane | 44.28 |
| branched alkane | 28.35 |
| cyclane | 16.79 |
| alkene | 0.13 |
| arene | 8.66 |
| others | 1.79 |
| Total | 100.00 |

Experimental conditions: cracking temperature is 850°C, and the dilution ratio of steam/total raw materials is 0.50 wt/wt.

Cracking furnace type: SRT-IV, available from ABB Lummus, Inc. of USA.

The results are as follows:

Ethylene yield: 30.16 wt%, propylene yield: 11.79 wt%, butadiene yield: 3.20 wt%.

### Example 4

Raw materials used in the experiment: refinery C4 + alkane-rich light hydrocarbon, each composition of which is listed in table 7 and 8 below respectively, wherein the refinery C4 comprises 15 wt% of the total raw materials.

**Table 7 Composition of refinery C4**

| Composition | Content (wt %) |
|---|---|
| propane | 3.11 |
| cyclopropane | 1.06 |
| propene | 0.01 |
| isobutane | 54.12 |
| n-butane | 9.37 |
| trans-2-butylene | 1.62 |
| cis-2-butylene | 3.55 |
| 1-butylene | 13.56 |
| isobutene | 12.38 |
| others | 1.22 |
| Total | 100.00 |

**Table 8 Composition of alkane-rich light hydrocarbon**

| Composition | Content (wt %) |
|---|---|
| linear alkane | 49.35 |
| branched alkane | 34.65 |
| cyclane | 15.14 |
| alkene | 0.18 |
| arene | 0.53 |
| others | 0.15 |
| Total | 100.00 |

Experimental conditions: cracking temperature is 850°C, and the dilution ratio of steam/total raw materials is 0.50 wt/wt.

Cracking furnace type: USC-80U, available from Stone & Webster, Inc. of USA.

The results are as follows:

Ethylene yield: 31.79 wt%, propylene yield: 11.82 wt%, butadiene yield: 3.36 wt%.

### Example 5

Raw materials used in the experiment: refinery C4 + alkane-rich light hydrocarbon, each composition of which is listed in table 9 and 10 below respectively, wherein the refinery C4 comprises 15 wt% of the total raw materials.

**Table 9 Composition of refinery C4**

| Composition | Content (wt %) |
|---|---|
| propane | 3.02 |
| cyclopropane | 1.46 |
| propene | 0.02 |
| isobutane | 54.12 |
| n-butane | 5.13 |
| trans-2-butylene | 1.75 |
| cis-2-butylene | 3.42 |
| 1-butylene | 10.21 |
| isobutene | 19.38 |
| others | 1.49 |
| Total | 100.00 |

**Table 10 Composition of alkane-rich light hydrocarbon**

| Composition | Content (wt %) |
|---|---|
| linear alkane | 45.33 |
| branched alkane | 26.48 |
| cyclane | 15.35 |
| alkene | 0.21 |
| arene | 9.53 |
| others | 3.10 |
| Total | 100.00 |

Experimental conditions: cracking temperature is 860°C, and the dilution ratio of steam/total raw materials is 0.50 wt/wt.

Cracking furnace type: SC-1, available from KBR, Inc. of USA.

The results are as follows:

Ethylene yield: 32.63 wt%, propylene yield: 12.41 wt%, butadiene yield: 3.06 wt%.

### Example 6

Raw materials used in the experiment: refinery C4 + alkane-rich light hydrocarbon, each composition of which is listed in table 11 and 12 below respectively, wherein the refinery C4 comprises 20 wt% of the total raw materials.

**Table 11 Composition of refinery C4**

| Composition | Content (wt %) |
|---|---|
| propane | 3.92 |
| cyclopropane | 1.23 |
| propene | 0.03 |
| isobutane | 46.36 |
| n-butane | 6.58 |
| trans-2-butylene | 4.10 |
| cis-2-butylene | 4.28 |
| 1-butylene | 12.06 |
| isobutene | 18.15 |
| others | 3.29 |
| Total | 100.00 |

**Table 12 Composition of alkane-rich light hydrocarbon**

| Composition | Content (wt %) |
|---|---|
| linear alkane | 44.28 |
| branched alkane | 28.35 |
| cyclane | 16.79 |
| alkene | 0.13 |
| arene | 8.66 |
| others | 1.79 |
| Total | 100.00 |

Experimental conditions: cracking temperature is 860°C, and the dilution ratio of steam/total raw materials is 0.60 wt/wt.

Cracking furnace type: SC-1, available from KBR, Inc. of USA.

The results are as follows:

Ethylene yield: 32.83 wt%, propylene yield: 11.41 wt%, butadiene yield: 3.19 wt%.

### Example 7

Raw materials used in the experiment: refinery C4 + alkane-rich light hydrocarbon, each composition of which is listed in table 13 and 14 below respectively, wherein the refinery C4 comprises 20 wt% of the total raw materials.

**Table 13 Composition of refinery C4**

| Composition | Content (wt %) |
|---|---|
| propane | 3.92 |
| cyclopropane | 1.23 |
| propene | 0.03 |
| isobutane | 46.36 |
| n-butane | 6.58 |
| trans-2-butylene | 4.10 |
| cis-2-butylene | 4.28 |
| 1-butylene | 12.06 |
| isobutene | 18.15 |
| others | 3.29 |
| Total | 100.00 |

**Table 14 Composition of alkane-rich light hydrocarbon**

| Composition | Content (wt %) |
|---|---|
| linear alkane | 44.28 |
| branched alkane | 28.35 |
| cyclane | 16.79 |
| alkene | 0.13 |
| arene | 8.66 |
| others | 1.79 |
| Total | 100.00 |

Experimental conditions: cracking temperature is 855°C, and the dilution ratio of steam/total raw materials is 0.50 wt/wt.

Cracking furnace type: USC-80U, available from Stone & Webster, Inc. of USA.

The results are as follows:

Ethylene yield: 31.51 wt%, propylene yield: 11.76 wt%, butadiene yield: 3.30 wt%.

### Comparative Example 1

Raw material used in the experiment: refinery C4, the composition of which is listed in table 15 below.

**Table 15 Composition of refinery C4**

| Composition | Content (wt %) |
|---|---|
| propane | 3.92 |
| cyclopropane | 1.23 |
| propene | 0.03 |
| isobutane | 46.36 |
| n-butane | 6.58 |
| trans-2-butylene | 4.10 |
| cis-2-butylene | 4.28 |
| 1-butylene | 12.06 |
| isobutene | 18.15 |
| others | 3.29 |
| Total | 100.00 |

Experimental conditions: cracking temperature is 860°C, and the dilution ratio of steam/total raw materials is 0.50 wt/wt.

Cracking furnace type: USC-80U, available from Stone & Webster, Inc. of USA.

The results are as follows:

Ethylene yield: 17.83 wt%, propylene yield: 13.41 wt%, butadiene yield: 2.19 wt%.

As apparent from the above results, the yield of ethylene is no more than 18% in the case where refinery C4 is singly used as the raw material, whereas the yield of ethylene is improved to be 30% or so when the mixtures of refinery C4 and the alkane-rich light hydrocarbon are used as the raw materials. Accordingly, notable economic benefits can be brought to the oil refinery.

Furthermore, it is experimentally observed that the cracking condition for the refinery C4 is similar to that for light hydrocarbon. And hence the known cracking furnace suitable for light hydrocarbon as raw material can also be applicable for the copyrolysis of the mixture of light hydrocarbon and refinery C4. No great fluctuations will be caused and thereby ensuring the smooth production in the cracking furnace.

Without wishing to be bound by any particular theory, the applicant believes the reason for the higher yield of ethylene through copyrolysis of refinery C4 and light hydrocarbon is as follows: hydrogen radical (H·) will have a strong impact on the formation of ethylene during the cracking process. With respect to the raw material of alkane-rich light hydrocarbon with high performance separately, more hydrogen radicals can be created during the cracking process, and the output and thus the yield of ethylene will be high. Moreover, in the atmosphere of thermal cracking, there are abundant hydrogen radicals. With respect to the raw material of refinery C4 with inferior performance separately, less hydrogen radicals will be produced during the cracking process due to high level of alkene therein, and the double bonds in alkene will have strong attracting effect on hydrogen radicals thereby resulting in less output and yield of ethylene. When these two kinds of raw materials are mixed together in proper proportion to undergo copyrolysis reaction, the abundant hydrogen radicals produced by the light hydrocarbon can be fully utilized to promote the cracking of C4, so that the cracking efficiency of C4 can be improved and thus the yield of ethylene will be raised. In other words, there is synergistic effect between C4 and light hydrocarbon during the copyrolysis, and such synergistic effect would cause the yield of ethylene to be greatly improved.

## Claims

1. A method of producing ethylene by using refinery C4 comprising:
• a refinery C4 as a first raw material which comprises 45 % - 55 % isobutane, 5 % - 10 % n-butane, 15 % - 20 % isobutene, 10 % - 15 % 1-butylene and 5 % - 10 % 2-butylene based on the total weight percentage of the refinery C4, the weight average molecular weight thereof being 55 - 60,
• an alkane-rich light hydrocarbon as a second raw material which comprises 40 % - 50 % linear alkane, 25 % - 35 % branched alkane, 15 % - 25 % cyclane, less than or equal to 1 % alkene and less than or equal to 10 % arene based on the total weight percentage of the alkane-rich light hydrocarbon, the weight average molecular weight thereof being 90 - 100;
the refinery C4 being in amount of 10 % to 30 % of the total weight percentage of the refinery C4 and alkane-rich light hydrocarbon;
introducing the mixture of said first raw material and said second raw material into the upper part of the convection section of a cracking furnace, after diluted with steam, passing the diluted mixture through the lower part of the convection section to enter into the radiation section of said cracking furnace,
distributing the diluted mixture to manifolds to carry out copyrolysis reaction therein so as to produce ethylene,
wherein the cracking temperature is 840 °C to 860 °C, and the dilution ratio of steam/total raw materials is 0.30 to 0.60 wt/wt.

2. The method according to claim 1, wherein the first raw material is in amount of 15 % - 20 % of the total raw materials.

3. The method according to claim 1, wherein said refinery C4 comprises 46.36 % isobutane, 6.58 % n-butane, 8.38 % 2-butylene, 12.06 % 1-butylene and 18.15 % isobutene based on the total weight percentage of said refinery C4.

4. The method according to claim 1, wherein said alkane-rich light hydrocarbon comprises 44.28 % linear alkane, 28.35 % branched alkane, 16.79 % cyclane, 0.13 % alkene and 8.66 % arene based on the total weight percentage of the alkane-rich light hydrocarbon,.

5. The method according to claim 1, wherein said cracking furnace is selected from the group consisting of USC furnace, SRT furnace and SC furnace.

## Patentansprüche

1. Verfahren zum Produzieren von Ethylen unter Verwendung einer C4-Fraktion aus der Raffinierung, umfassend
- eine C4-Fraktion aus der Raffinierung als erstes Ausgangsmaterial, welches umfaßt: 45 % bis 55 % Isobutan, 5 % bis 10 % n-Butan, 15 % bis 20 % Isobuten, 10 % bis 15 % 1-Butylen und 5 % bis 10 % 2-Butylen, bezogen auf die Gesamt-Gewichtsprozent-Menge der C4-Fraktion aus der Raffinierung, wobei das Gewichtsmittel des Molekulargewichts davon 55 bis 60 beträgt;
- einen an Alkan reichen leichten Kohlenwasserstoff als zweites Ausgangsmaterial, welcher umfaßt: 40 % bis 50 % lineares Alkan, 25 % bis 35 % verzweigtes Alkan, 15 % bis 25 % Cyclan, weniger als oder gleich 1 % Alken und weniger als oder gleich 10 % Aren, bezogen auf die Gesamt-Gewichtsprozent-Menge des an Alkan reichen leichten Kohlenwasserstoffs, wobei das Gewichtsmittel des Molekulargewichts davon 90 bis 100 beträgt;
wobei die C4-Fraktion aus der Raffinierung mengenmäßig 10 % bis 30 % der Gesamt-Gewichtsprozent-Menge von C4-Fraktion aus der Raffinierung und an Alkan reichem leichtem Kohlenwasserstoff beträgt;
Einleiten der Mischung des ersten Ausgangsmaterials und des zweiten Ausgangsmaterials in den oberen Teil der Konvektionszone des Cracking-Ofens, nachdem sie mit Dampf verdünnt wurde, Durchleiten der verdünnten Mischung durch den unteren Teil der Konvektionszone des Cracking-Ofens und dann Eintreten in die Strahlungszone des Cracking-Ofens;
Verteilen der verdünnten Mischung auf Verteiler unter Durchführen einer CoPyrolyse-Reaktion darin unter Produzieren von Ethylen;
worin die Cracking-Temperatur 840 °C bis 860 °C beträgt und das Verdünnungs-Verhältnis Dampf/Gesamt-Ausgangsmaterialien 0,30 bis 0,60 (Gewicht/Gewicht) beträgt.

2. Verfahren nach Anspruch 1, worin das erste Ausgangsmaterial in einer Menge von 15 % bis 20 % der Gesamtmenge der Ausgangsmaterialien vorliegt.

3. Verfahren nach Anspruch 1, worin die C4-Fraktion aus der Raffinierung umfaßt: 46,36 % Isobutan, 6,58 % n-Butan, 8,38 % 2-Butylen, 12,06 % 1-Butylen und 18,15 % Isobuten, bezogen auf die Gesamt-Gewichtsprozent-Menge der C4-Fraktion aus der Raffinierung.

4. Verfahren nach Anspruch 1, worin der an Alkan reiche leichte Kohlenwasserstoff umfaßt: 44,28 % lineares Alkan, 28,35 % verzweigtes Alkan, 16,79 % Cyclan, 0,13 % Alken und 8,66 % Aren, bezogen auf die Gesamt-Gewichtsprozent-Menge des an Alkan reichen leichten Kohlenwasserstoffs.

5. Verfahren nach Anspruch 1, worin der Cracking-Ofen gewählt ist aus der Gruppe, die besteht aus USC-Ofen, SRT-Ofen und SC-Ofen.

## Revendications

1. Procédé de production d'éthylène en utilisant des produits de raffinerie en C₄ comprenant :
• un produit de raffinerie en C₄ comme première matière première qui comprend 45 % à 55 % d'isobutane, 5 % à 10 % de n-butane, 15 % à 20 % d'isobutène, 10 % à 15 % de 1-butylène et 5 % à 10 % de 2-butylène sur la base du pourcentage total en poids du produit de raffinerie en C₄, son poids moléculaire moyen en poids étant de 55 à 60,
• un hydrocarbure léger riche en alcane comme seconde matière première qui comprend 40 % à 50 % d'alcane linéaire, 25 % à 35 % d'alcane ramifié, 15 % à 25 % de cyclane, 1 % ou moins d'alcène et 10 % ou moins d'arène sur la base du pourcentage total en poids de l'hydrocarbure léger riche en alcane, son poids moléculaire moyen en poids étant de 90 à 10 ;
le produit de raffinerie en C₄ se trouvant en une quantité de 10 % à 30 % du pourcentage total en poids du produit de raffinerie en C4 et de l'hydrocarbure léger riche en alcane ;
l'introduction du mélange de ladite première matière première et de ladite seconde matière première dans la partie supérieure de la section de convection d'un fourneau de craquage, après avoir été dilué avec l'écoulement, le passage du mélange dilué à travers la partie inférieure de la section de convection pour entrer dans la section d'irradiation dudit fourneau de craquage,
la distribution du mélange dilué à des rampes de distribution pour conduire une réaction de copyrolyse à l'intérieur afin de produire de l'éthylène,
dans lequel la température de craquage est de 840°C à 860°C, et le rapport de dilution des matières premières écoulement/totales est de 0,30 à 0,60 en pds/pds.

2. Procédé selon la revendication 1, dans lequel la première matière première se trouve en une quantité de 15 % à 20 % des matières premières totales.

3. Procédé selon la revendication 1, dans lequel ledit produit de raffinerie en C₄ comprend 46,36 % d'isobutane, 6,58 % de n-butane, 8,38 % de 2-butylène, 12,06 % de 1-butylène et 18,15 % d'isobutène sur la base du pourcentage total en poids dudit produit de raffinerie en C₄.

4. Procédé selon la revendication 1, dans lequel ledit hydrocarbure léger riche en alcane comprend 44,28 % d'alcane linéaire, 28,35 % d'alcane ramifié, 16,79 % de cyclane, 0,13 % d'alcène et 8,66 % d'arène sur la base du pourcentage total en poids de l'hydrocarbure léger riche en alcane.

5. Procédé selon la revendication 1, dans lequel ledit four de craquage est choisi parmi le groupe constitué du fourneau de craquage USC, fourneau de craquage SRT et fourneau de craquage SC.
